Europäisches Patentamt

European Patent Office

Office européen des brevets

(19)

(11) Publication number: **0 097 459**
**B1**

(12) # EUROPEAN PATENT SPECIFICATION

(45) Date of publication of patent specification: **17.12.86**

(51) Int. Cl.⁴: **A 01 C 1/00, C 12 N 1/04**

(21) Application number: **83303346.7**

(22) Date of filing: **09.06.83**

(54) Inoculation of seeds with freeze-dried microorganisms.

(30) Priority: **11.06.82 US 387306**

(43) Date of publication of application:
**04.01.84 Bulletin 84/01**

(45) Publication of the grant of the patent:
**17.12.86 Bulletin 86/51**

(84) Designated Contracting States:
**BE CH DE FR GB IT LI NL SE**

(56) References cited:
**EP-A-0 064 720**
**FR-A-1 068 699**
**FR-A-1 350 860**
**FR-A-1 482 817**
**FR-A-2 469 861**
**US-A-3 168 796**

(73) Proprietor: **MORAN SEEDS, INC.**
**1211 Avenue of the Americas**
**New York, N.Y. 10036 (US)**

(72) Inventor: **Cull, Steven W.**
**916 Castroville Blvd.**
**Castroville California (US)**
Inventor: **Messenger, Derinda L.**
**229 Lerwick Drive**
**Monterey California (US)**

(74) Representative: **De Minvielle-Devaux, Ian
Benedict Peter et al
CARPMAELS & RANSFORD 43, Bloomsbury
Square
London WC1A 2RA (GB)**

Courier Press, Leamington Spa, England.

## Description

The field of art to which this invention pertains is the treatment of agricultural and horticultural seeds with microorganisms to enhance productivity.

For a number of years, plant seeds have been inoculated with microorganisms to improve the plants growing from the seeds. For example, nitrogen-fixing bacteria have been used to treat seeds of legumes so that the resulting plants will be less dependent upon soil nitrates for growth.

Inoculation techniques require contacting the seeds with microorganisms. Frequently an inoculum liquid, such as a bacterial culture dispersed in water, is used for such contacting. In addition to the microorganisms, sometimes auxiliary, beneficial materials, such as surface-tension depressants and growth-promoting materials also have been included in the liquid inoculum. Organisms on carriers, such as peat, charcoal and the like, have been dispersed in a dilute, aqueous syrup to provide inoculum liquids. Materials recognized as detrimental and destructive to the microorganisms, seeds and plants have been excluded from the inoculum liquids.

Inoculum liquids have been applied by a variety of techniques, including slurry, spray and immersion treatments. Seeds have been inoculated by spraying inoculum liquids on seeds which are tumbled in a barrel or by slurrying a mixture of seeds and the inoculum liquid.

In seed inoculation processes, it is customary to try to produce uniformly inoculated seeds having a minimum effective dose of organisms per seed. This minimum effective dose is the smallest number of microorganisms that will produce the desired effect in each and every host plant. This seed population is dependent on the particular seeds and particular organism bein employed.

In U.S. Patent No. 2,954,643, a fluidized bed is used to inoculate seeds. The inoculating liquid containing the microorganisms is introduced into a bed of seeds which are maintained in a fluid state by an inert gas.

U.S. Patent No. 3,054,219 describes inoculating seeds with an excess of microorganisms in order to be sure that a minimum effective dose is retained on the seed.

U.S. Patent No. 2,313,057, U.S. Patent No. 2,995,867 and U.S. Patent No. 4,149,869 describe pelleting the seed, i.e., coating the seed with materials which include those which are beneficial to the plant.

A different inoculation technique is described in U.S. Patent No. 3,499, 748. Granules of plaster of Paris are treated with cultures of *Azotobacter* or *Rhizobium* bacteria. These granules are then mixed with seeds and the mixture is sown on the soil.

In U.S. Patent No. 4,155,737, microorganisms are incorporated into a polymer gel. The gel is crushed into small particles and is sown with the seeds.

Commercialization of seed-applied microorganisms has been impeded by the difficulty of maintaining microbial viability on dry seed, particularly desiccation-susceptible bacteria of the genera *Achromobacter*, *Arthrobacter*, *Azotobacter* and *Pseudomonas* [Brown, *Ann. Rev. Phytopath*, 12:181-97 (1974); Kloepper and Schroth, *Phytopath*, 71:590-92 (1981)].

U.S. Patent No. 3, 168,796 relates to the application to seeds of freeze-dried bacteria and a carrier by means of dry mixing to produce a substantially dry coating on the seeds.

The present invention provides a process of inoculating agricultural seeds with beneficial freeze-dried microorganisms, characterised by applying said freeze-dried microorganisms to the seeds along with a binder applied from an aqueous solution or dispersion and drying the seeds.

The present invention also provides an agricultural seed coated with a freeze-dried beneficial microorganism, characterised in that said freeze-dried microorganism is in a coating of a water-soluble or water-dispersible binder, said seed having a moisture content of 3 to 8% by weight based on the weight of the coated seed.

In carrying out this invention, beneficial microorganisms are freeze-dried, are applied to agricultural seeds with a binder and are dried to a moisture content of 3—8 weight percent depending on plant species at a temperature below about 45°C. In an alternate process, the freeze-dried microorganism can be applied to the agricultural seeds along with a binder and a filler. In another variation of the process, the agricultural seeds, after being coated or pelleted with binder and filler, can be inoculated with the freeze-dried microorganism and additional binder.

Microorganisms useful in this invention are those which have been described as telluric microorganisms having a favorable rhizospheric effect on plants. Microorganisms of this type and their effects on plants are described in U.S. Patent No. 4,155,737 which is hereby incorporated by reference.

Examples of beneficial microorganisms include, but are not limited to, *Rhizobium*, *Enterobacter*, *Spirillum*, *Bacillus*, *Pseudomonas*, *Azotobacter*, *Arthrobacter*, *Achromobacter* and *Actimomycetes*. Examples of fungi include *Penicillium*, *Trichoderma*, *Chaetomium*, and those forming ectomycorrhizae and vesicular-arbuscular mycorrhizae. Additional microorganisms are mycoparastic fungi, nematode-trapping fungi as well as avirulent isolates of pathogenic fungi or bacteria which may control diseases caused by related plant pathogens.

Seeds which can be coated by the process of this invention include all agricultural or horticultural seeds. The term "agricultural seeds" is used generically herein to include both agricultural and horticultural seeds. Examples of such seeds include lettuce, broccoli, cauliflower, cabbage, celery, carrot, onion, radish, tomato, corn, oats, legumes, grasses and ornamental flowers.

Freeze-drying of the microorganisms can be conducted in any of the various freeze-dryers which are commercially available. An example of a freeze-dryer is Freeze-Dryer 5 (Labconco, Kansas City, Mo. 64132).

The freeze-drying process can be carried out at a condenser temperature of less than about −40°C, preferably about −76°C to about −65°C. Useful pressure ranges are less than 33.3 Pa (0.25 Torr), preferably less than 3.33 Pa (0.025 Torr).

In carrying out the freeze-drying operation, the culture containing the microorganisms can be freeze-dried directly. However, it is preferred to remove the excess medium from the cells, e.g., by centrifugation or filtration, and then suspend the cells in a liquid. Water can be used as the suspending liquid. However, it is preferred to suspend the cells in a cryoprotectant. Examples of cryoprotectants include glycerol, skim milk, sucrose and dimethyl sulfoxide. A preferred cryoprotectant is one made from aqueous 0.1 molar (M) sucrose, 0.05 M glycerol, 0.07 M sodium benzoate and 0.01 M magnesium sulfate. The cells are suspended in the cryoprotectant on a volume basis of about 3:1 to about 1:1 cryoprotectant to cells, preferably about 2:1. After being freeze-dried, the microorganisms are recovered as a dry powder. The freeze-dried product generally contains in the order of $10^{12}$ colony forming units per gram (cfu/g). Freeze-drying of microorganisms and cryoprotectants used in the freeze-drying process are described in detail in "American Type Culture Collection Methods" of the American Type Culture Collection, Rockville, Md., which is hereby incorporated by reference.

The freeze-dried microorganisms are applied to the seeds uniformly, generally with a small amount of binder and, preferably with binder and filler. This application can be conducted in a rotating pan or cement mixer or in a fluidized bed. When the microorganisms are to be applied directly to the seed, the seeds and microorganism are tumbled together so that the microorganism powder coats the seed uniformly. A small amount of binder is then sprayed on the tumbling seeds to adhere the microorganisms to the seed. The inoculated seeds are then dried with forced air at a temperature of about 20°C to about 45°C to a moisture content of about 3 to about 8% by weight based on the weight of the inoculated seed.

Preferably, the freeze-dried microorganisms are applied to pelleted seeds, i.e., seeds coated with binder and filler. In a pelleting process, the seeds are tumbled in a rotating apparatus while binder and filler are alternately added until the proper size pelleted seed is obtained. The pelleted seeds are then force air dried at a temperature of about 30° to about 45°C to a moisture content of about 3 to about 8% by weight based on the weight of the pelleted seed. In this process the microorganisms can be added to the seed mixture at any time during the process. Preferably, the microorganisms are added so that they are about in the middle of the coating.

The microorganisms can also be applied to previously pelleted seeds. This is done by tumbling the pelleted seeds, adding the freeze-dried microrganism with a light spray of binder and then drying the inoculated pelleted seed as previously described.

The amount of inoculum which is applied to the seed will vary with the type of organism, seed and soil. Generally this amount of inoculum will be in the order of about $10^2$ to about $10^8$ cfu/seed. In order to relate cfu/seed to weight, $10^6$ cfu/seed translates to about 1—5 micrograms of inoculum per seed. This converts to a rate of 0.56—1.12 grams inoculum per kilogram of lettuce seed or 0.06—0.11 g/kg radish seed.

Binders which can be used in the inoculating and coating process are dilute solutions of water soluble or water dispersible synthetic polymers or natural gum products. Examples of such binders are methyl cellulose, methylethyl cellulose, gum arabic, guar, casein, hydrolyzed gelatin, polyvinyl alcohol, polyoxyethylene glycols, starch, hydroxyethyl cellulose, karaya, tragacanth, polyvinyl acetate and the like.

Useful fillers are organic and inorganic powders which can be used to build up a coating on the seed without adversely affecting the seeds. Examples of such filler materials are bentonite, titanium dioxide, charcoal, vermiculite, sand, diatomaceous earth, clays, silica, mica and the like.

Other ingredients, such as fertilizers and humectants, can be added in the coating process if desired.

In first attempts to apply microorganisms to pelleted seeds, an aqueous suspension of a *Pseudomonas* strain of microorganism was sprayed during pelleting of vegetable seeds with a polyvinyl alcohol binder and a diatomaceous earth filler. The microorganisms survived the pelleting operation but were killed during the drying process regardless of the drying time or temperature which was varied from 20°C to 45°C. Viable bacteria could not be recovered from pelleted seeds containing less than 30% moisture. The moisture content of pelleted seeds is generally kept in the range of about 3—8% by weight and, preferably, about 4—6%.

When the bacterium was prepared in a methyl cellulose/diatomaceous earth formulation, no survival was noted when the pelleted seed was dried at 43°C. A *Pseudomonas* spp., in an inoculum prepared by M. N. Schroth's Laboratory [Phytopath 71:590-92 (1981)], failed to survive pelleting and drying.

A xanthan gum/diatomaceous earth inoculum was prepared according to Kloepper and Schroth [Phytopath 71:590-92 (1981)] giving approximately $3 \times 10^8$ cfu/g. This inoculum was applied to radish seeds during pelleting in an amount which should have been measured as $2 \times 10^6$ cfu/seed. However, even after drying the pelleted seed at 20°C in a forced air dryer, the population on the seeds declined to $3\times10^4$ cfu/seed. Also, the coatings on the seeds dried at this low temperature cracked and fell off the seeds.

Example 1

A two day King's B broth shake culture of *Pseudomonas* strain MCB-1A was centrifuged and the cells washed twice with distilled water. Washed cells from 2 liter broth cultures were suspensed in 100 ml distilled water and were freeze-dried at −76°C, 33.3 Pa (0.25 Torr) for 8 hours. The resulting powder was assayed and found to contain $5\times10^9$ cfu/g. Two grams of this powder were applied to 28 g broccoli seeds (~9750 seeds) during pelleting with polyvinyl alcohol and diatomaceous earth. A sample of the pelleted seed was assayed before drying and was found to have a population of $1\times10^6$ cfu/seed, the calculated theoretical amount. After drying at 20°C in a forced air dryer to a moisture content of 5—6 weight %, the bacteria population on the dry seed was determined to be $5\times10^5$ cfu/seed. When similar coated seeds weré dried at a temperature of 43°C, the same amount of bacteria was found on the seeds.

Example 2

A 150 ml culture of *Pseudomonas* strain MCB-170 (more desiccation susceptible than MCB-1A) was centrifuged and the cells were washed twice with distilled water. The washed cells ($5\times10^{12}$ cells) were suspended in 6 ml of an aqueous solution containing 0.1 M sucrose, 0.05 M glycerol, 0.01 M $MgSO_4$ and 0.07 M sodium benzoate. After being freeze-dried at −76°C., 33.3 Pa (0.25 Torr) for 8 hours, the dried powder was found to contain $3\times10^{11}$ cfu/g. 0.77 g. of this powder was applied to 7 g of lettuce seeds during pelleting with polyvinyl alcohol and diatomaceous earth. After drying at 43°C in a forced air dryer to a moisture content of 5—6% by weight, the population on the dry seed was determined to be $4.7\times10^5$ cfu/seed.

Example 3

Freeze-dried *Pseudomonas* strain MCB-170, prepared as described in Example 2, and suspended in an aqueous cryoprotectant solution as described in Example 2, was applied to lettuce seeds during pelleting with polyvinyl alcohol and diatomaceous earth in an amount sufficient to provide $3\times10^7$ cfu/seed. Three runs were made with the inoculum being applied near the seed in one run, in the middle of the coating in another run and near the surface of the coating in the third run. The final pellet diameter was 3—3.5 mm. After drying at 43°C with forced air to a moisture content of 6—7 weight %, the seed assay revealed seed population of $1.4—2.0\times10^4$ cfu/seed for each run. The inoculated seeds were planted in both field soil and a sterile potting mix. Differences in root populations were very small with a slightly higher population being noted with the seed inoculated in the middle of the coating.

Example 4

A 14 liter culture of *Pseudomonas* strain MCB-170 (very susceptible to desiccation) was centrifuged and the recovered cells were suspended in 560 ml cryoprotectant as described in Example 2 and freeze-dried at a condenser temperature of −65°C, pressure of 3.33 Pa (0.025 Torr) for 20 hours. The freeze-dried inoculum was added to carrot seed during pelleting with polyvinyl alcohol and diatomaceous earth in an amount sufficient to provide $1.5\times10^5$ cfu/seed. Three runs were made with the inoculum being applied near the seed in one run, in the middle of the coating in another run, and near the surface of the coating in the third run. The final pellet diameter was about 2.0 to 2.4 mm. After drying at 43°C with forced air to a moisture content of 4—5%, the seed assays revealed seed populations of $3.6\times10^1$ cfu/seed (next to the seed), $1.3\times10^3$ cfu/seed (in middle of coating) and $1.9\times10^2$ cfu/seed (exterior of the coating).

Example 5

Freeze-dried inoculum of *Pseudomonas* strain MCB-172, prepared as described above, was applied to lettuce seed during pelleting. The inoculum was also applied to previously pelleted and dried lettuce seed by applying the freeze-dried powder to the pellets, spraying the pellets with a polyvinyl alcohol binder and drying the pellets at 43°C. No differences in process survival or root population were observed.

Example 6

Lettuce seed was treated with freeze-dried inoculum of *Pseudomonas* strain MCB-114 by applying the inoculum with a light spray of a poly(vinyl alcohol) binder followed by a short period of drying at 43°C. Survival of the microorganisms was as good as, or better than, the same strain applied to lettuce seed during pelleting. A comparison of root populations from seed inoculated by pelleting vs. raw seed treatment revealed no differences.

Example 7

The death rate of freeze-dried bacteria on pelleted seed was examined. Broccoli and radish seeds were pelleted with freeze-dried *Pseudomonas* strain MCB-1A. Pelleted seed was stored in sealed plastic containers at 4°, 14°, or 22°C and assayed periodically over a period of 34 weeks. Initial seed populations of $10^7$ cfu/seed declined to $10^5$ cfu/seed in 2.5—3 weeks at 22°C, 4—5 weeks at 14°C and 34 weeks at 4°C. For strain MCB-1A on either broccoli or radish seed stored at 4°C, the seed population declines to about 10% of the original in 18 weeks and to about 1% of the original in 34 weeks.

Example 8

The fungus *Penicillium oxalicum* strain MCF-6 was freeze-dried in a suspension of 20% skim milk as described in American Type Culture Collection Methods. Lettuce seed pellets were successfully inoculated with this fungus. A strain of *Trichoderma* spp (MCF-20) was also successfully processed and pelleted as above.

Example 9

One liter of King's B Broth (containing in g/l:Difco proteose peptone No. 3, 20; glycerol, 12.6; $MgSO_4 \cdot 7H_2O$, 1.5 and $K_2HPO_4$, 1.5) was inoculated with *Pseudomonas* strain MCB-121 and incubated on a rotary shaker at 200 rpm at 20—22°C for 3 days. The cells were separated from the medium by centrifugation (10,000×g. for 20 minutes at 5°C). The cells were then suspended in 40 ml cryoprotectant (0.1 M sucrose and 0.05 M glycerol) and freeze-dried with a vacuum of 6.6 Pa (0.05 Torr) and condenser temperature of −76°C for 6 hours. The dried powder was recovered and found to weigh 4.54 g. To determine the bacterial population in the inoculum, 0.01 g. was rehydrated in 9.9 ml sterile distilled water for 30 minutes in a shaking test tube. Dilutions were plated on semi-solid King's B agar (King's B Broth supplemented with 15 g/l agar). The population determined in this way was $6.4 \times 10^{11}$ cfu/g.

The remaining inoculum (4.53 g.) was added to seed during pelleting. 0.9 Kg (two pound) runs of radish variety Cherry Belle (germination 90%; 173,668 seeds/Kg (78,775 seeds/lb;)) were pelleted using a binder of 15% poly(vinyl alcohol) Gelvatol (40—20 (Monsanto Corp.) and 1% glycerol and a diatomaceous earth/cellulose fiber mixture (Fibracel 8A, Johns-Manville Corp.) filler. Drying was conducted for 1.5 hours at 43°C in a forced air dryer.

Theoretical seed population was determined as follows:

$$\text{cfu/seed:} \quad \frac{4.53 \text{ g.} \times 6.4 \times 10^{11} \text{ cfu/g.}}{\dfrac{(2 \text{ lb. seed}) \times (78,775 \text{ seeds/lb.})}{0.9 \text{ Kg seed} \times 173,668 \text{ seeds/Kg}}} = 1.8 \times 10^7 \text{ cfu/seed}$$

Actual seed population was determined to be $5 \times 10^5$ cfu/seed by shaking 10 seeds in 9.9 ml sterile distilled water for 30 minutes followed by plating dilutions on King's B agar.

The pelleted seeds were planted in 3 different field soils from the Salinas Valley in pots in a greenhouse. Plants were grown for 30—35 days at 12°—17°C night temperatures and 18°—22°C day temperatures. Radish root weights from the inoculated seeds grown in the three soils were 119%, 119% and 151% of those from non-inoculated pelleted seeds grown under the same conditions.

**Claims**

1. A process of inoculating agricultural seeds with beneficial freeze-dried microorganisms, characterised by applying said freeze-dried microorganisms to the seeds along with a binder applied from an aqueous solution or dispersion and drying the seeds.

2. The process of claim 1 wherein the coating includes a filler.

3. The process of claim 1 wherein the coated seed is dried at a temperature of 20°C to 45°C to a moisture content of 3 to 8% by weight based on the weight of the coated seed.

4. The process of claim 3 wherein the temperature is 30°C to 45°C and the moisture content is 4 to 6% by weight.

5. The process of claim 1 wherein the microorganisms are freeze-dried at a temperature below −40°C at a pressure less than 33.3 Pa (0.25 Torr).

6. The process of claim 5 wherein the temperature is about −76°C to about −65°C, and the pressure is less than 3.33 Pa (0.025 Torr).

7. The process of any of claims 1—6 wherein the binder is a synthetic polymer or natural gum product.

8. The process of claim 7 wherein the binder is selected from methyl cellulose, methylethyl-cellulose, gum arabic, guar, casein, hydrolyzed gelatin, polyvinyl alcohol, polyoxyethylene glycols, starchhydroxyethyl cellulose, karaya, tragacanth and polyvinyl acetate.

9. An agricultural seed coated with a freeze-dried beneficial microorganism, characterised in that said freeze-dried microorganism is in a coating of a water-soluble or water-dispersible binder, said seed having a moisture content of 3 to 8% by weight based on the weight of the coated seed.

10. The seed of claim 9 having a moisture content of 4 to 6% by weight.

11. The seed of claim 7 or 8 having a coating which comprises a filler.

12. The seed of any of claims 9—11 wherein the binder is a synthetic polymer or natural gum product.

13. The seed of claim 12 wherein the binder is selected from methyl cellulose, methylethyl cellulose, gum arabic, guar, casein, hydrolyzed gelatin, polyvinyl alcohol, polyoxyethylene glycols, starch, hydroxyethyl cellulose, karaya, tragacanth and polyvinyl acetate.

**Patentansprüche**

1. Verfahren zum Impfen von landwirtschaftlichem Saatgut bzw. Samen mit geeigneten gefriergetrockneten Mikroorganismen, gekennzeichnet durch

— Aufbringen der gefriergetrockenten Mikro-organismen auf das Saatgut zusammen mit einem Bindemittel, angewandt in einer wäßrigen Lösung oder einer Dispersion, und
— Trocknen des Saatguts.

2. Verfahren nach Anspruch 1, worin die Beschichtung ein Füllmittel einschließt.

3. Verfahren nach Anspruch 1, worin das beschichtete Saatgut bei einer Temperatur von 20°C bis 45°C auf einen Feuchtigkeitsgehalt von 3 bis 8 Gew.-%, bezogen auf das Gewicht des beschichten Saatguts, getrocknet wird.

4. Verfahren nach Anspruch 3, worin die Temperatur 30°C bis 45° C und der Feuchtigkeitsgehalt 4 bis 6 Gew.-% beträgt.

5. Verfahren nach Anspruch 1, worin die Mikroorganismen bei einer Temperatur unterhalb −40°C bei einem Druck von weniger als 33,3 Pa (0,25 Torr) gefriergetrocknet werden.

6. Verfahren nach Anspruch 5, worin die Temperatur etwa −76°C bis etwa −65°C und der Druck weniger als 3,33 Pa (0,025 Torr) beträgt.

7. Verfahren nach einem der Ansprüche 1 bis 6, worin das Bindemittel ein synthetisches Polymer oder ein natürliches Gummiprodukt ist.

8. Verfahren nach Anspruch 7, worin das Bindemittel aus Methylcellulose, Methylethylcellulose, Gummiarabikum, Guar-Gummi, Kasein, hydrolysierter Gelatine, Polyvinylalkohol, Polyoxyethylenglykolen, Stärke, Hydroxyethylcellulose, Sterkulien- bzw. Karayagummi, Tragantgummi und Polyvinylacetat gewählt wird.

9. Landwirtschaftliches Saatgut, beschichtet mit einem gefriergetrockneten, geeigneten Mikroorganismus, dadurch gekennzeichnet, daß der gefriergetrocknete Mikroorganismus in einer Beschichtung aus einem wasserlöslichen oder wasserdispergierbaren Bindemittel ist, wobei das Saatgut einen Feuchtigkeitsgehalt von 3 8 Gew.-%, bezogen auf das Gewicht des beschichteten Saatguts, besitzt.

10. Saatgut nach Anspruch 9 mit einem Feuchtigkeitsgehalt von 4 bis 6 Gew.-%.

11. Saatgut nach Anspruch 7 oder 8 mit einer Beschichtung, die ein Füllmittel umfaßt.

12. Saatgut nach einem der Ansprüche 9 bis 11, worin das Bindemittel ein synthetisches Polymer oder ein natürliches Gummiprodukt ist.

13. Saatgut nach Anspruch 12, worin das Bindemittel aus Methylcellulose, Methylethylcellulose, Gummiarabikum, Guar-Gummi, Kasein, hydrolysierter Gelatine, Polyvinylalkohol, Polyoxyethylenglykolen, Stärke, Hydroxyethylcellulose, Sterkulien- bzw. Karayagummi, Tragantgummi und Polyvinylacetat gewählt wird.

**Revendications**

1. Procédé pour inoculer des semences agricoles à l'aide de microorganismes lyophilisés intéressants, caractérisé en ce qu'il consiste à appliquer lesdits microorganismes lyophilisés en même temps qu'un liant appliqué à partir d'une solution ou d'une dispersion aqueuse, et à sécher les semences.

2. Procédé selon la revendication 1, dans lequel l'enrobage comprend une charge.

3. Procédé selon la revendication 1, dans lequel la semence enroé est séchée à une température de 20—45°C jusqu'à une teneur en humidité de 3—8% en poids par rapport au poids de la semence enrobée.

4. Procédé selon la revendication 3, dans lequel la température est de 30 à 45°C et la teneur en humidité est de 4 à 6% en poids.

5. Procédé selon la revendication 1, dans lequel les microorganismes sont lyophilisés à une température inférieure à −40°C sous une pression inférieure à 33,3 Pa (0,25 Torr).

6. Procédé selon la revendication 5, dans lequel la température est d'environ −76 à environ −65°C, et la pression est inférieure à 3,33 Pa (0.025 Torr).

7. Procédé selon l'une quelconque des revendications 1 à 6, dans lequel le liant est un polymère synthétique ou un produit de gomme naturelle.

8. Procédé selon la revendication 7, dans lequel le liant est choisi entre la méthylcellulose, la méthyléthylcellulose, la gomme arabique, la gomme de guar, la caséïne, la gélatine hydrolysée, le poly(alcool vinylique), les polyoxyéthylèneglycol, l'amidon, l'hydroxyéthylcellulose, la gomme de karaya, l'adragante et le poly(acétate de vinyle).

9. Semence agricole enrobée d'un microorganisme lyophilisé intéressant, caractérisée en ce que ledit microorganisme lyophilisé se trouve dans un enrobage d'un liant soluble ou dispersible dans l'eau, ladite semence ayant une teneur en humidité de 3 à 8% en poids par rapport au poids de la semence enrobée.

10. Semence selon la revendication 9, ayant une teneur en humidité de 4 à 6% en poids.

11. Semence selon la revendication 7 ou 8, ayant un enrobage comprenant une charge.

12. Semence selon l'une quelconque des revendications 9—11, dans laquelle le liant est un polymère synthétique ou un produit de gomme naturelle.

13. Semense selon la revendication 12, dans laquelle le liant eso choisi entre la méthylcellulose, la méthyléthylcellulose, la gomme arabique, la gomme de guar, la caséïne, la gélatine hydrolysée, le poly(alcool vinylique), les polyoxyéthylèneglycols, l'amidon, l'hydroxyéthylcellulose, la gomme de karaya, la gomme adragante et le poly(acétate de vinyle).